# EUROPÄISCHE PATENTSCHRIFT

(11) **EP 1 162 195 B1**
(45) Veröffentlichungstag und Bekanntmachung des Hinweises auf die Patenterteilung: **03.05.2006**
(21) Anmeldenummer: 01112086.2
(22) Anmeldetag: 29.05.2001
(51) Int. Cl.: C07C 303/24, C07C 305/10

(54) **Mischungen von Schwefelsäureestern**
Mixtures of sulphuric acid esters
Mélanges d' esters d'acide sulfurique

(30) Priorität: 07.06.2000 DE 10028224
(43) Veröffentlichungstag der Anmeldung: 12.12.2001
(73) Patentinhaber: Lanxess B.V., 6710 BA Ede (NL)
(72) Erfinder: Vogt, Uwe, Dr., 40789 Monheim (DE)
(74) Vertreter: Herbold, Matthias

(56) Entgegenhaltungen:
- EP-A- 0 593 392
- DE-A- 1 940 178
- DE-A- 19 848 894

## Beschreibung

Die vorliegende Erfindung betrifft neue Mischungen von Schwefelsäureestern und spezielle Schwefelsäureester, Verfahren zu ihrer Herstellung und ihre Verwendung als Färbereihilfsmittel.

Bei der Färbung von Textilien werden Egalisiermittel als Hilfsmittel eingesetzt. Bei diesen Egalisiermitteln handelt es sich um grenzflächenaktive Substanzen, welche die Aufgabe haben, die zu färbende Faser oder Fasermischung gut zu benetzen, das Durchfärben der Fasern zu fördern und ein zu schnelles, unegales Aufziehen der Farbstoffe beim Färbeprozess zu verhindern. Durch den Einsatz der Egalisiermittel kann somit ein gleichmäßiges und einheitliches Färbeergebnis erreicht werden.

Das Färben von stickstoffhaltigen Fasermaterialien, insbesondere von Wolle, erfolgt geeigneterweise mit anionischen Farbstoffen, wie z.B. Säurefarbstoffen, 1:1-Metallkomplexfarbstoffen, 1:2-Metallkomplexfarbstoffen, Chromfarbstoffen oder deren Mischungen.

Aus dem Stand der Technik sind bereits verschiedenste Egalisiermittel für die Färbung von Textilmaterialien, insbesondere Wolle, mit den angeführten Farbstoffen bekannt. Bekannt ist beispielsweise die Verwendung von Phosphorsäureestern (DE-A-16 19 372, OE 19848894 A1 und DE-A-19 46 058).

Bekannt ist ferner aus der DE-A-19 40 178 die Verwendung von Polyalkylenglykoletherketten aufweisenden Ethansulfonsäureverbindungen oder deren Quatemisierungsprodukten als Egalisiermittel für das Färben stickstoffhaltiger Fasermaterialien mit Säurefarbstoffen oder 1:2-Metallkomplexfarbstoffen. Die Polyalkylenglykoletherketten aufweisenden Ethansulfonsäureverbindungen besitzen dabei folgende Strukturformel

R₁R₂N-(CH₂-CHR₃-O)ₘ-CH₂-CH₂-SO₃M

wobei
- R₁: für einen C₁₂-C₂₂-Alkyl-, einen C₁₂-C₂₂-Alkenyl- oder C₁₂-C₂₂-Cycloalkylrest,
- R₂: für CH₃ oder eine der Gruppierungen -(CH₂-CHR₃-O-)ₙH oder -(CH₂-CHR₃-O)ₙ-CH₂-CH₂-SO₃M,
- R₃: für H, CH₃,C₂H₅ oder Phenyl steht,
- M: ein Kation darstellt und die Summe aus m+n eine Zahl von 5-70 ist, wobei mindestens 80 % der im Molekül enthaltenen Alkylenoxid-Einheiten Ethylenoxid-Einheiten sind.

In EP-A-0 593 392 wird die Verwendung von Verbindungen der Formel 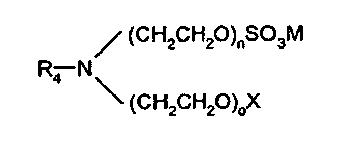 wobei
R₄ ein C₁₂-C₂₄-Alkyl- oder Alkenylrest, X H oder SO₃M bedeutet und M für H, Alkalimetall oder Ammonium steht und n+o =2-20 ist, als Egalisierhilfsmittel beim Färben von Fasermaterialien aus natürlichem oder synthetischem Polyamid in wässriger Flotte beschrieben.

Auch in Wool Dyeing (Hrsgeber: D. M Lewis; 1992, Society of Dyers and Colourists) werden sulfatierte ethoxylierte Amine als amphotere Hilfsmittel für das Färben von Wolle beschrieben.

Die Aufgabe der vorliegenden Erfindung bestand darin, neue Substanzen zur Verfügung zu stellen, die für den Einsatz als Egalisiermittel bei der Färbung von Textilmaterialien, insbesondere Wolle, geeignet sind.

Gelöst wird diese Aufgabe durch Mischungen von Schwefelsäureestern der allgemeinen Formel 1, 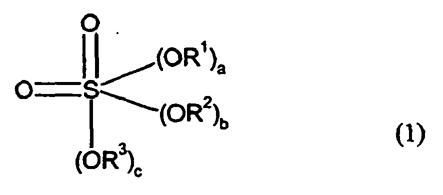 worin
- R¹: ein aliphatischer Rest mit 1-30 C-Atomen ist,
- R²: ein Rest der allgemeinen Formel 2 ist, 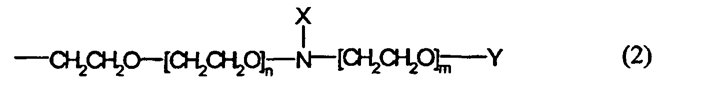 wobei
n eine ganze Zahl von 0 - 30,
m eine ganze Zahl von 1 - 29,
X ein aliphatischer Rest mit 4-24 C-Atomen und
Y H oder SO₂(OM) ist, wobei M unabhängig voneinander für Wasserstoff, Alkalimetall-, Ammonium-, Mono-, Di-, Tri- oder Tetra- (C₁-C₆-Alkyl)-ammonium oder Mono-, Di-, Tri- oder Tetra-(C₂-C₆₋Alkanol)-ammoniumionen steht,
R³ einen Rest der allgemeinen Formel 3 darstellt, 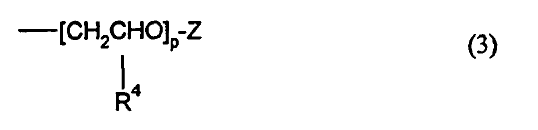 wobei
p eine ganze Zahl von 4 - 35 ist,
R⁴ H, Methyl, Ethyl, Phenyl oder Mischungen aus H und Methyl bedeutet und
Z H, Methyl, Ethyl oder SO₂(OM) ist, wobei M unabhängig voneinander für Wasserstoff, Alkalimetall-, Ammonium-, Mono-, Di-, Tri- oder Tetra-(C₁-C₆-Alkyl)-ammonium- oder Mono-, Di-, Tri- oder Tetra-(C₂-C₆-Alkanol)-ammoniumionen steht, und
a, b und c gleich oder verschieden und 0, 1 oder 2 sind und a+b+c=2 ist, erhältlich durch Umsetzung von Sulfurylchlorid mit einer Mischung der Alkohole R¹OH, R²OH und R³OH, wobei R¹, R² und R³ die für die allgemeine Formel 1 genannten Bedeutungen besitzen, Y jedoch ausschließlich für Wasserstoff und Z entweder für Wasserstoff, Methyl oder Ethyl steht.

In den Schwefelsäureestern der Formel 1 steht der Rest R¹ für einen aliphatischen Rest mit 1 bis 30 C-Atomen, bevorzugt handelt es sich um geradkettige oder verzweigte Alkyl- oder Alkenylreste mit 1-30 C-Atomen. Beispiele für geradkettige oder verzweigte C₁-C₈-Alkylgruppen sind Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Etylpentyl, 1-Propylbutyl, 2-Ethylhexyl und n-Octyl.

Beispiele für geradkettige oder verzweigte C₉-C₃₀-Alkylgruppen sind n-Nonyl, iso-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl und n-Docosyl.

Die Bedeutung der Alkenylgruppe umfasst geradkettige und verzweigte C₁-C₃₀-Alkenylgruppen mit 1, 2 oder 3 Doppelbindungen. Vorzugsweise handelt es sich um geradkettige oder verzweigte C₄-C₃₀-Alkenyl-, insbesondere C₈-C₂₀-Alkenylgruppen. Beispiele für Alkenylgruppen sind Heptenyl, Octenyl, Nonenyl, Decenyl, Undecenyl, Tridecenyl, Tetradecenyl, Pentadecenyl, Hexadecenyl, Heptadecenyl, Octadecenyl, Nonadecenyl, Hexadecadienyl, Hexadecatrienyl, Octadecadienyl sowie Octadecatrienyl.

Die Bedeutung des Alkyl- bzw. Alkenylrestes für den Rest R¹ umfasst auch Mischungen verschiedener Alkyl- bzw. Alkenylgruppen, wie sie z.B. in natürlichen Fetten und Ölen, z.B. Kokosöl, Palmkernöl, Sojaöl, Rüböl, Olivenöl, Sonnenblumenöl, Baumwollsaatöl, Erdnussöl, Leinöl, Rapsöl oder Talgfett vorkommen.

Im Rest R² der allgemeinen Formel 2 steht X für einen aliphatischen Rest mit 4-24 Kohlenstoffatomen. Als solche aliphatischen Reste kommen geradkettige und verzweigte Alkyl- und Alkenylreste mit 4-24 C-Atomen, bevorzugt 12-24 und besonders bevorzugt 16-22 C-Atomen in Betracht. Insbesondere steht X für einen Dodecyl-, Tetradecyl-, Hexadecyl-, Octadecyl-, Eicosyl-, Docosyl-, Octadecenyl-oder Abietylrest.

Die Bedeutung des Alkyl- bzw. Alkenylrestes für X umfasst dabei ebenfalls Mischungen verschiedener Alkyl- bzw. Alkenylgruppen, wie sie z.B. in natürlichen Fetten und Ölen, z.B. Kokosöl, Palmkernöl, Sojaöl, Rüböl, Olivenöl, Sonnenblumenöl, Baumwollsaatöl, Erdnussöl, Leinöl, Rapsöl oder Talgfett vorkommen.

Die Gruppe Y steht im Rest R² der allgemeinen Formel 2 für H oder SO₂(OM). Für M kommen als Kationen unabhängig voneinander Wasserstoff, Alkalimetall-, bevorzugt Natrium- oder Kalium, Ammonium-, Mono-, Di-, Tri- oder Tetra- (C₁-C₆₋Alkyl)-ammonium- oder Mono-, Di-, Tri- oder Tetra- (C₂-C₆-Alkanol)-ammoniumionen zum Einsatz. Letztere leiten sich inbesondere von Mono-, Di- oder Triethanolamin ab.

Der Index n steht im Rest R² der allgemeinen Formel 2 für eine ganze Zahl von 0 bis 30, bevorzugt 0 bis 10, insbesondere 0 bis 5. Der Index m steht im Rest R² der allgemeinen Formel 2 für eine ganze Zahl von 1 bis 29, bevorzugt 1 bis 10, insbesondere 1 bis 5.

Im Rest R³ der allgemeinen Formel 3 steht die Gruppe Z für H, Methyl, Ethyl oder SO₂(OM). Vorzugsweise steht Z für H oder SO₂(OM). Für M kommen als Kationen unabhängig voneinander Wasserstoff, Alkalimetall-, bevorzugt Natrium- oder Kalium-, Ammonium-, Mono-, Di-, Tri- oder Tetra- (C₁-C₆-Alkyl)-ammonium- oder Mono-, Di-, Tri- oder Tetra- (C₂-C₆-Alkanol)-ammoniumionen in Frage. Letztere leiten sich inbesondere von Mono-, Di- oder Triethanolamin ab.

Der Rest R⁴ in der allgemeinen Formel 3 bedeutet Wasserstoff, Methyl, Ethyl, Phenyl oder auch Mischungen aus Wasserstoff und Methyl. Bevorzugt steht R⁴ für Wasserstoff oder Methyl. Bei Mischungen aus Wasserstoff und Methyl können maximal 80% des Gesamtwertes von R⁴ für Methyl und minimal 20 % des Gesamtwertes von R⁴ für Wasserstoff stehen.

Der Index p steht in der allgemeinen Formel 3 für eine ganze Zahl von 4 bis 35, bevorzugt 9 bis 22.

Bevorzugt sind Mischungen von Schwefelsäureestern der allgemeinen Formel 1, wobei
- R¹: ein aliphatischer Rest mit 4-30 C-Atomen ist,
- R²: einen Rest der allgemeinen Formel 2 darstellt, worin
n eine ganze Zahl von 0-10,
m eine ganze Zahl von 1 - 10,
X ein aliphatischer Rest mit 12-24 C-Atomen und
Y H oder SO₂(OM) ist, wobei M unabhängig voneinander für Wasserstoff, Alkalimetall-, Ammonium-, Mono-, Di-, Tri- oder Tetra-(C₁-C₆-Alkyl)-ammonium- oder Mono-, Di-, Tri- oder Tetra- (C₂-C₆₋Alkanol)-ammoniumionen steht,
- R³: einen Rest der allgemeinen Formel 3 darstellt, worin
m eine ganze Zahl von 3 - 35,
R⁴ H oder Methyl und
Z H, Methyl, Ethyl oder SO₂(OM) ist, wobei M unabhängig voneinander für Wasserstoff, Alkalimetall-, Ammonium-, Mono-, Di-, Tri- oder Tetra-(C₁-C₆-Alkyl)-ammonium- oder Mono-, Di-, Tri- oder Tetra-(C₂-C₆-Alkanol)-ammoniumionen steht, und
a, b und c gleich oder verschieden und 0, 1 oder 2 sind und a+b+c=2 ist.

Besonders bevorzugt sind Mischungen von Schwefelsäureestern der allgemeinen Formel 1, wobei
- R¹: ein aliphatischer Rest mit 8-20 C-Atomen ist,
- R²: einen Rest der allgemeinen Formel 2 darstellt, worin
n eine ganze Zahl von 0-5,
m eine ganze Zahl von 1-5,
X ein aliphatischer Rest mit 16-22 C-Atomen und
Y H ist,
- R³: einen Rest der allgemeinen Formel 3 darstellt, worin
p eine ganze Zahl von 9-22,
R¹ H und
Z H ist und
a, b und c gleich oder verschieden und 0, 1 oder 2 sind und a+b+c=2 ist.

Gegenstand der Erfindung ist ferner ein Verfahren zur Herstellung der Mischungen von Schwefelsäureestern der allgemeinen Formel 1 durch Umsetzung von Sulfurylchlorid mit einer Mischung der Alkohole R¹OH, R²OH und R³OH, wobei in diesen Alkoholen R¹, R² und R³ die für die allgemeine Formel 1 genannten Bedeutungen besitzen, Y jedoch ausschließlich für Wasserstoff und Z entweder für Wasserstoff, Methyl oder Ethyl steht.

In einer geeigneten Ausführungsform des erfindungsgemäßen Verfahrens setzt man insgesamt 3 mol einer Mischung der Alkohole R¹OH, R²OH und R³OH mit 1,5 bis 2,5 mol, bevorzugt mit 1,5 bis 1,8 mol Sulfurylchlorid um.

Die drei Alkohole R¹OH, R²OH und R³OH können dabei in folgenden Mengenverhältnissen zueinander verwendet werden:

| | |
|---|---|
| R¹OH | 10 - 40, bevorzugt 30 - 40 mol % |
| R²OH | 20 - 80, bevorzugt 30 - 40 mol % und |
| R³OH | 10 - 40, bevorzugt 30 - 40 mol %, |

wobei die Summe der Mengen der drei Alkohole immer 100 mol% beträgt.

Üblicherweise legt man zunächst die Alkohole R¹OH, R²OH und R³OH vor, erwärmt diese Mischung unter Aufschmelzen der Alkohole und gibt bei 40 bis 60°C das Sulfurylchlorid zu. Bei dieser Zugabe kommt es zu einer Erhöhung der Temperatur, in der Regel auf mindestens 80°C. Anschließend wird die Reaktionsmischung bei erhöhter Temperatur, bevorzugt ungefähr 100°C, nachgerührt. Durch Verminderung des Drucks ist es möglich, die bei der Umsetzung entstandene HCl aus dem Reaktionsprodukt zu entfernen. Es hat sich ferner bewährt, anschließend durch Zugabe einer Base wie Natronlauge oder Diethanolamin einen pH-Wert von 5 bis 7 einzustellen.

Die Alkohole R¹OH sind allgemein zugänglich. Die Herstellung der Alkohole R²OH kann nach dem Fachmann bekannten Methoden erfolgen und ist beispielsweise in der DE-A-1 940 178 beschrieben. Zunächst lagert man 2 bis 60 Mol Ethylenoxid an ein aliphatisches Amin X-NH₂ an, wobei X die oben angeführte Bedeutungen besitzt. Als aliphatische Amine haben sich insbesondere Dodecyl-, Tetradecyl-, Hexadecyl-, Octadecyl-, Eicosyl-, Docosyl, Octadecenyl- oder Abietylamin bewährt.

Bevorzugte Beispiele sind ferner Umsetzungsprodukte aus 1 mol Talgfettamin mit 2 mol, 5 mol oder 10 mol Ethylenoxid.

Die Veresterung der endständigen OH Gruppe im Rest R², so dass Y anschließend für SO₂(OM) steht, ist im Rahmen der Umsetzung der Alkohole mit Sulfurylchlorid möglich, sofern mit einem Überschuss an Sulfurylchlorid gearbeitet wird.

Die Alkohole R³ OH sind allgemein zugänglich. Bevorzugte Beispiele für R³OH sind Triethylenglykol und Polyethylenglykole mit mittleren Molekulargewichten von 200 bis 1 500 g/mol oder Monomethyl- bzw. Monoethylether dieser Verbindungen.

Die Veresterung der endständigen OH Gruppe im Rest R³, so dass Z anschließend für SO₂(OM) steht, ist im Rahmen der Umsetzung der Alkohole mit Sulfurylchlorid möglich, sofern mit einem Überschuss an Sulfurylchlorid gearbeitet wird.

Natürlich sind auch alle anderen dem Fachmann bekannten Herstellungsmethoden geeignet, solange sie zu den erfindungsgemäßen Mischungen von Schwefelsäureestern der allgemeinen Formel 1 führen.

Das sich im Rahmen der erfindungsgemäßen Umsetzung bildende Reaktionsprodukt stellt eine Mischung von verschiedenen Schwefelsäureestern der allgemeinen Formel 1 dar. Sofern gewünscht, ist es auch möglich, aus diesen Mischungen einzelne Schwefelsäureester durch geeignete und dem Fachmann geläufige Methoden abzutrennen.

Gegenstand der Erfindung sind auch spezielle Schwefelsäureester der allgemeinen Formel 1, worin
- R¹, R² und R³: die für die erfindungsgemäßen Mischungen der Schwefelsäureester genannten Bedeutungen besitzen und
- a, b und c: gleich oder verschieden und 0 oder 1 sind und a+b+c=2 ist.

Bevorzugt sind solche Schwefelsäureester der allgemeinen Formel 1, worin
- R¹, R² und R³: die für die bevorzugten Mischungen der Schwefelsäureester genannten Bedeutungen besitzen und
- a, b und c: gleich oder verschieden und 0 oder 1 sind und a+b+c=2 ist.

Besonders bevorzugt sind solche Schwefelsäureester der allgmeinen Formel 1, worin
- R¹, R² und R³: die für die besonders bevorzugten Mischungen der Schwefelsäureester genannten Bedeutungen besitzen und
- a, b und c: gleich oder verschieden und 0 oder 1 sind und a+b+c=2 ist.

Die erfindungsgemäßen Mischungen der Schwefelsäureester der Formel I bzw. die speziellen oben definierten Schwefelsäureester liegen in der Regel bei Raumtemperatur in fester, wachsartiger Form vor. Im Bereich von 30 bis 60°C beginnen sie zu schmelzen. Um diese Mischungen der Schwefelsäureester bzw. die speziellen Schwefelsäureester in eine flüssige und damit anwendungsfreundlichere, weil leichter dosierbare Form zu überführen, empfiehlt es sich, organische oder wässrig-organische Formulierungen herzustellen.

Gegenstand der Erfindung sind daher auch organische oder wässrig-organische Formulierungen enthaltend 25-70 Gew.-% der Mischungen der Schwefelsäureester der allgemeinen Formel 1 oder der speziellen oben definierten Schwefelsäureester. Als organische Lösungsmittel sind solche geeignet, die sich gegenüber den Schwefelsäureestern inert verhalten und wassermischbar sind. Bevorzugt werden Mono-, Di-oder Oligoethylenglykole, -propylenglykole oder -ethylen/ propylenglykole, deren Mono- oder Diether oder Mischungen daraus eingesetzt. Insbesondere wird Butyldiglykol oder Methyldiglykol verwendet. Zur Herstellung der Formulierungen der Mischungen der Schwefelsäureester der allgemeinen Formel 1 kann das Reaktionsgemisch des erfindungsgemäßen Verfahrens ohne weitere Aufarbeitung eingesetzt werden.

Gegenstand der Erfindung ist ferner die Verwendung der Mischungen der Schwefelsäureester der Formel 1 oder der speziellen oben definierten Schwefelsäureester oder deren jeweiligen organischen oder wässrig-organischen Formulierungen als Färbereihilfsmittel beim Färben von stickstoffhaltigen Fasermaterialien, bevorzugt Wolle.

Die Mischungen der Schwefelsäureester der allgemeinen Formel 1 oder die speziellen oben definierten Schwefelsäureester bzw. deren jeweilige organische oder wässrig-organische Formulierungen werden den Färbeflotten zugesetzt. Das Färben der stickstoffhaltigen Textilmaterialien, insbesondere Wolle, in Gegenwart der erfindungsgemäßen Mischungen der Schwefelsäureester der Formel 1 oder der speziellen oben definierten Schwefelsäureester kann dabei so durchgeführt werden, dass man das Färbegut zunächst in eine auf 40- 50°C erwärmte Färbeflotte einbringt, die den Farbstoff, die Mischungen der Schwefelsäureester der allgemeinen Formel 1 oder die speziellen oben definierten Schwefelsäureester und Säuren, wie z.B. Essigsäure, enthält. Anschließend erhitzt man das Färbebad allmählich auf 100 bis 130°C und hält es so lange auf dieser Temperatur, bis es erschöpft ist. Es ist ebenso möglich, das Färbegut mit einer wässrigen Flotte, die nur die Mischung der Schwefelsäureester der Formel 1 bzw. die speziellen Schwefelsäureester und Säure enthält, kurze Zeit bei 40 bis 50°C vorzubehandeln und erst dann dieser Flotte bei Temperaturen zwischen 40 bis 98°C die Farbstoffe zuzusetzen, anschließend die Temperatur des Färbebades allmählich auf 100 - 130°C zu steigern und bis zur Baderschöpfung auf dieser Temperatur zu halten.

Als Farbstoff kommen die für das Färben von stickstoffhaltigen Fasermaterialien, insbesondere Wolle, geeigneten anionischen Farbstoffe, z.B. Säurefarbstoffe, 1:1-Metallkomplexfarbstoffe, 1:2-Metallkomplexfarbstoffe oder Chromfarbstoffe sowie deren Mischungen in Betracht.

Die Mengen, in denen die erfindungsgemäßen Mischungen oder die speziellen Schwefelsäureester den Färbebädern zugesetzt wird, können in weiten Grenzen schwanken. Sie lassen sich durch Vorversuche leicht ermitteln. Im allgemeinen haben sich Mengen von 0.25-3.0 Gew.-%, vorzugsweise 0.5 bis 1.0 Gew.-%, bezogen auf das Gewicht des Färbegutes, bewährt.

Das Färbeverfahren eignet sich für alle mit den aufgeführten Farbstoffen anfärbbaren stickstoffhaltigen Fasermaterialien, insbesondere für natürliche Polyamide, wie Wolle und Seide, für synthetische Polyamide, wie Hexamethylendiadipat, Poly-ε-caprolactam und Poly-ω-aminoundecansäure, für basisch modifizierte Polyacrylnitrile sowie für deren Mischungen untereinander oder mit anderen Fasermaterialien, wie solchen aus nativer und regenerierter Cellulose, Polyacrylnitril, Polyurethanen oder Polyestern. Die genannten anfärbbaren Fasermaterialien können dabei in den unterschiedlichsten Verarbeitungsformen vorliegen, beispielsweise als Flocke, Kammzug, texturierte Fäden, Spinnkabel, Garn, Gewebe, Gewirke oder Vliese.

Mit Hilfe der erfindungsgemäßen Mischungen der Schwefelsäureester der allgemeinen Formel 1 bzw. der speziellen oben definierten Schwefelsäureester gelingt es, diese stickstoffhaltigen Textilmaterialien sehr gleichmäßig zu färben. Neben ihrer hervorragenden Qualität als Färbereihilfsmittel zeigen die Mischungen der Schwefelsäureester der allgemeinen Formel 1 sowie die speziellen Schwefelsäureester überraschenderweise auch eine gute biologische Abbaubarkeit.

### Beispiele:

### Beispiel 1 (Herstellung einer erfindungsgemäßen Mischung der Schwefelsäureester)

Ein Gemisch aus 27.0 g (0.1 Mol) Stearylalkohol, 40.0g (0.1 Mol) Polyethylenglykol mit einem mittleren Molgewicht von 400 und 48.8 g (0.1 Mol) eines Umsetzungsproduktes aus 1 Mol Talgfettamin mit 5 Mol Ethylenoxid wird bei 55°C vorgelegt und innerhalb von 10 min mit 20.2 g (0.15 Mol) Sulfurylchlorid versetzt. Die Temperatur steigt dabei auf 90°C. Unter einem leichten Stickstoffstrom lässt man anschließend 4 Stunden bei 100°C nachrühren. Es folgt eine weitere Stunde Nachrührzeit unter Vakuum (20 mbar). Man lässt auf 50°C abkühlen und stellt den Ansatz durch vorsichtige Zugabe von 45%iger Natronlauge auf einen pH-Wert von 5-6 ein. Nach dem Abkühlen auf Raumtemperatur erstarrt das Reaktionsprodukt zu einer wachsartigen Masse.

### Beispiel 2 (Herstellung einer erfindungsgemäßen Mischung der Schwefelsäureester)

Es wird wie in Beispiel 1 angegeben verfahren, aber Diethanolamin anstatt der Natronlauge zum Neutralisieren des Ansatzes benutzt. Man erhält nach dem Abkühlen eine wachsartige Masse.

### Beispiel 3 (Herstellung einer erfindungsgemäßen Mischung der Schwefelsäureester)

Es wird wie in Beispiel 1 angegeben verfahren, aber auf das Neutralisieren verzichtet. Man erhält man eine wachsartige Masse, die, 5%ig in destillierten Wasser gelöst, einen pH-Wert von 2.8 zeigt.

### Beispiel 4 (Herstellung einer erfindungsgemäßen Mischung der Schwefelsäureester)

In Analogie zu Beispiel 1 wird die Umsetzung von 24.2 g (0.1 Mol) Hexadecylalkohol, 40.0 g (0.1 Mol) Polyethylenglykol mit einem mittleren Molgewicht von 400 und 48.8 g (0.1 Mol) eines Umsetzungsproduktes aus 1 Mol Talgfettamin mit 5 Mol Ethylenoxid durchgeführt. Nach dem Erkalten resultiert ein wachsartiges Produkt.

### Beispiel 5 (Herstellung einer erfindungsgemäßen Mischung der Schwefelsäureester)

Es wird wie in Beispiel 4 angegeben verfahren, aber Diethanolamin anstatt der Natronlauge zum Neutralisieren des Ansatzes benutzt. Man erhält ebenfalls eine wachsartige Masse.

### Beispiel 6 (Herstellung einer flüssigen Zubereitung der Mischung der Schwefelsäureester aus Beispiel 2)

100 g der erfindungsgemäßen Mischung aus Beispiel 2 werden bei 50°C mit 100 g Butyldiglykol bis zur klaren Lösung verrührt. Anschließend lässt man abkühlen.

### Beispiel 7 (Herstellung einer flüssigen Zubereitung der Mischung der Schwefelsäureester aus Beispiel 5)

30 g der erfindungsgemäßen Mischung aus Beispiel 5 werden bei 50°C mit 40 g Butyldiglykol und 30 g entmineralisierten Wasser bis zur klaren Lösung verrührt. Anschließend lässt man abkühlen.

### Beispiel 8 (Anwendung)

30 g Wollkammgarn werden in einem Zirkulationsfärbeapparat (Typ Color Star der Firma Mathis) wie folgt gefärbt. Prozentangaben beziehen sich hierbei auf das Warengewicht. Die eingesetzen ISOLANE® sind Farbstoffe der DyStar Deutschland GmbH & Co. KG.

### 1. Färbung

Flottenumpumpung: Wechselseitig 4 min von innen nach außen und 2 min von außen nach innen

| | |
|---|---|
| Flotte: ISOLAN Gelb K-GLN 250% | 0.3% |
| ISOLAN Bordo R 220% | 0.15% |
| ISOLAN Grau K-BRLS 200% | 0.3% |
| Natriumsulfat | 5% |
| Essigsäure 60% | 3% |
| Mischung gemäß Beispiel 1 | 0.5% |

pH-Wert der Flotte: 4.5
Flottenverhältnis: 1:20
Starttemperatur: 50°C
10 min Vorlaufzeit bei 50°C
Aufheizen auf 98°C (Aufheizgeschwindigkeit 2°C/min)
30 min bei 98°C halten
Abkühlen auf 70°C (Abkühlgeschwindigkeit 2°C/min)
5 min bei 70°C halten
Ablassen der Flotte

### 2. Spülen

Flottenumpumpung: Wechselseitig 4 min von innen nach außen und 2 min von außen nach innen
Temperatur: 40°C
Spüldauer: 12 min
Ablassen der Spülflotte
Auffüllen neuer Spülflotte
Temperatur: 60°C
Spüldauer: 12 min
Ablassen der Spülflotte

### 3. Absaugen und Trocknen der Kammgarnspule

12 Stunden bei 50°C

Aus dem gefärbten Wollkammgarn wird ein Strickstück Innen-Mitte-Außen hergestellt. Bewertet wird der Farbtonablauf des Strickstückes, d.h. die Farbtonabweichung zwischen dem inneren-, mittleren- und äußeren Bereich der Kammgarnspule.

Es resultiert ein Strickstück mit einwandfreiem Farbtonablauf.

### Beispiel 9 (Anwendung)

Wird die in Beispiel 8 beschriebene Färbung mit folgender Flotte

| | |
|---|---|
| ISOLAN Gelb S-GL | 0.3% |
| ISOLAN Rot S-RL | 0.3% |
| ISOLAN Grau S-GL | 0.3% |
| Natriumsulfat | 5% |
| Essigsäure 60% | 3% |
| Mischung gemäß Beispiel 5 | 0.5% |

durchgeführt, so resultiert ebenfalls ein Strickstück mit einwandfreiem Farbtonablauf

### Beispiel 10 und 11 (Vergleichsbeispiele)

Wird die in den Beispielen 8 und 9 beschriebene Färbung ohne die erfindungsgemäße Mischung von Schwefelsäureestern als Hilfsmittel durchgeführt, so resultiert aus dem gefärbten Wollkammgarn ein Strickstück, das einen sehr deutlichen Innen-Mitte-Außen-Effekt, d.h. einen starken Farbtonunterschied innerhalb des Strickstückes zeigt.

## Patentansprüche

1. Mischung von Schwefelsäureestern der allgemeinen Formel 1, 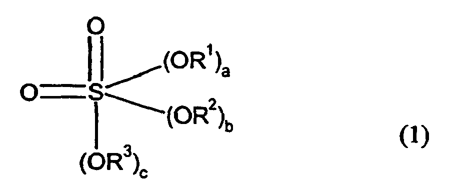 worin
R¹ ein aliphatischer Rest mit 1-30 C-Atomen ist,
R² einen Rest der allgemeinen Formel 2 darstellt, 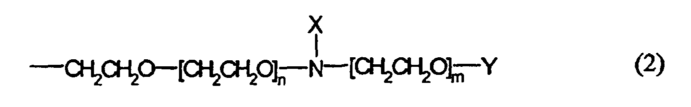 wobei
n eine ganze Zahl von 0-30
m eine ganze Zahl von 1-29
X ein aliphatischer Rest mit 4-24 C-Atomen und
Y H oder SO₂(OM) ist, wobei M unabhängig voneinander für H, Alkalimetall, Ammonium, Mono-, Di-, Tri- oder Tetra- (C₁₋C₆-Alkyl)-ammoniumionen oder Mono-, Di-, Tri- oder Tetra-(C₂-C₆-Alkanol)-ammoniumionen steht,
R³ einen Rest der allgemeinen Formel 3 darstellt, 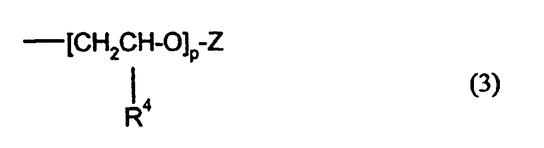 wobei
p eine ganze Zahl von 4 - 35 ist,
R⁴ H, Methyl, Ethyl oder Phenyl oder Mischungen aus H und Methyl bedeutet und
Z H, Methyl, Ethyl oder SO₂(OM) ist, wobei M unabhängig voneinander für H, Alkalimetall, Ammonium, Mono-, Di-, Tri-oder Tetra-(C₁-C₆-Alkyl)-ammoniumionen oder Mono-, Di-, Tri- oder Tetra- (C₂-C₆-Alkanol)-ammoniumionen steht, und
a, b und c gleich oder verschieden und 0,1 oder 2 sind und a+b+c=2 ist,
erhältlich durch Umsetzung von Sulfurylchlorid mit einer Mischung der Alkohole R¹OH, R²OH und R³OH, wobei in diesen Alkoholen R¹, R² und R³ die für die allgemeine Formel 1 genannten Bedeutungen besitzen, Y jedoch ausschließlich für Wasserstoff und Z entweder für Wasserstoff, Methyl oder Ethyl steht.

2. Mischungen von Schwefelsäureestern nach Anspruch 1, **dadurch gekennzeichnet, dass**
R¹ ein aliphatischer Rest mit 4-30 C-Atomen ist,
R² ein Rest der allgemeinen Formel 2 ist, worin
n eine ganze Zahl von 0-10,
m eine ganze Zahl von 1 - 10,
X ein aliphatischer Rest mit 12-24 C-Atomen und
Y H oder SO₂(OM) ist, wobei M unabhängig voneinander für H, Alkalimetall, Ammonium, Mono-, Di-, Tri- oder Tetra-(C₁-C₆₋Alkyl)-ammoniumionen oder Mono-, Di-, Tri- oder Tetra- (C₂₋C₆-Alkanol)-ammoniumionen steht und
R³ einen Rest der allgemeinen Formel 3 darstellt, worin
m eine ganze Zahl von 3 - 35,
R⁴ H oder Methyl und
Z H, Methyl, Ethyl oder SO₂(OM) ist, wobei M unabhängig voneinander für H, Alkalimetall, Ammonium, Mono-, Di-, Tri-oder Tetra-(C₁-C₆-Alkyl)-ammoniumionen oder Mono-, Di-, Tri- oder Tetra-(C₂-C₆-Alkanol)-ammoniumionen steht, und
a, b und c gleich oder verschieden und 0, 1 oder 2 ist und a+b+c=2 ist.

3. Mischungen von Schwefelsäureestern nach Anspruch 1, **dadurch gekennzeichnet, dass**
R¹ ein aliphatischer Rest mit 8-20 C-Atomen ist,
R² ein Rest der allgemeinen Formel 2 ist, worin
n eine ganze Zahl von 0-5,
m eine ganze Zahl von 1-5,
X ein aliphatischer Rest mit 16-22 C-Atomen und
Y H ist,
R³ einen Rest der allgemeinen Formel 3 darstellt, worin
p eine ganze Zahl von 9-22,
R⁴ H und
Z H ist und
a, b und c gleich oder verschieden und 0, 1 oder 2 ist und a+b+c=2 ist.

4. Verfahren zur Herstellung von Mischungen von Schwefelsäureestern nach einem oder mehreren der Ansprüche 1-3, **dadurch gekennzeichnet, dass** man eine Mischung der Alkohole R¹OH, R²OH und R³OH mit Sulfurylchlorid umsetzt, wobei in diesen Alkoholen R¹, R² und R³ die für die allgemeine Formel 1 angegebenen Bedeutungen besitzen, Y jedoch ausschließlich für Wasserstoff und Z entweder für Wasserstoff, Methyl oder Ethyl stehen.

5. Verfahren nach Anspruch 4, **dadurch gekennzeichnet, dass** man 3 mol der Mischung der Alkohole R¹OH, R²OH und R³OH mit 1,5-2,5 mol, bevorzugt 1,5-1,8 mol Sulfurylchlorid umsetzt.

6. Verfahren nach Anspruch 4 oder 5, **dadurch gekennzeichnet, dass** die Alkohole R¹OH, R²OH und R³OH in folgenden Mengenverhältnissen eingesetzt werden
| | |
|---|---|
| R¹OH | 10 - 40, bevorzugt 30 - 40 mol % |
| R²OH | 20 - 80, bevorzugt 30 - 40 mol % und |
| R³OH | 10 - 40, bevorzugt 30 - 40 mol %, |
wobei die Summe der Mengen der drei Alkohole immer 100 mol% beträgt.

7. Schwefelsäureester der allgemeinen Formel 1 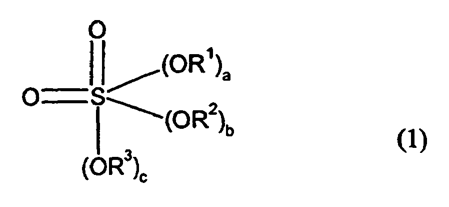 wobei
R¹, R² und R³ die für die Mischungen der Schwefelsäureester in Anspruch 1 genannten Bedeutungen besitzen und
a, b und c gleich oder verschieden und 0 oder 1 sind und a+b+c=2 ist.

8. Schwefelsäureester nach Anspruch 7, **dadurch gekennzeichnet, dass**
R¹, R² und R³ die für die Mischungen der Schwefelsäureester in Anspruch 2 genannten Bedeutungen besitzen und
a, b und c gleich oder verschieden und 0 oder 1 sind und a+b+c=2 ist.

9. Schwefelsäureester nach Anspruch 7, **dadurch gekennzeichnet, dass**
R¹, R² und R³ die für die Mischungen der Schwefelsäureester in Anspruch 3 genannten Bedeutungen besitzen und
a, b und c gleich oder verschieden und 0 oder 1 sind und a+b+c=2 ist.

10. Organische oder wässrig-organische Formulierungen enthaltend 25 - 70 Gew.-% der Mischungen der Schwefelsäureester nach einem oder mehreren der Ansprüche 1-3 oder der Schwefelsäureester nach einem oder mehreren der Ansprüche 7-9.

11. Organische oder wässrig-organische Formulierungen nach Anspruch 10, **dadurch gekennzeichnet, dass** als organische Lösungsmittel Mono-, Di- oder Oligoethylenglykole, -propylenglykole oder -ethylen/propylenglykole, deren Mono- oder Diether oder Mischungen daraus, insbesondere Butyldiglykol oder Methyldiglykol eingesetzt werden.

12. Verwendung der Mischungen der Schwefelsäureester nach einem oder mehreren der Ansprüche 1-3, der Schwefelsäureester nach einem oder mehreren der Ansprüche 7-9 oder der Formulierungen nach Anspruch 10 oder 11 als Färbereihilfsmittel beim Färben von stickstoffhaltigen Fasermaterialien, bevorzugt Wolle.

13. Verwendung nach Anspruch 12, wobei die Färbung mit Säurefarbstoffen, 1:1-Metallkomplexfarbstoffen, 1:2-Metallkomplexfarbstoffen, Chromfarbstoffen oder deren Mischungen durchgeführt wird.

## Claims

1. Mixture of sulphuric esters of the general formula 1 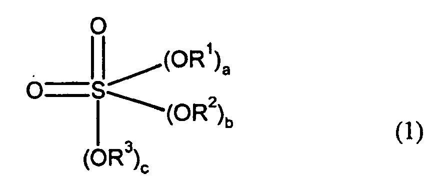 where
R¹ is an aliphatic radical having 1-30 carbon atoms,
R² is a radical of the general formula 2 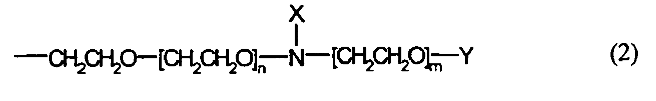 where
n is an integer from 0-30
m is an integer from 1-29
X is an aliphatic radical having 4-24 carbon atoms and
Y is H or SO₂(OM), where M represents H, alkali metal, ammonium, mono-, di-, tri- or tetra-(C₁-C₆-alkyl)ammonium ions or mono-, di-, tri- or tetra-(C₂-C₆-alkanol)ammonium ions,
R³ is a radical of the general formula 3 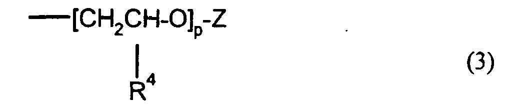 where
p is an integer from 4 to 35,
R⁴ is H, methyl, ethyl or phenyl or mixtures of H and methyl and
Z is H, methyl, ethyl or SO₂(OM), where M independently represents H, alkali metal, ammonium, mono-, di-, tri- or tetra-(C₁-C₆-alkyl)ammonium ions or mono-, di-, tri- or tetra-(C₂₋C₆-alkanol)ammonium ions, and
a, b and c are identical or different and 0, 1 or 2 and a+b+c=2,
obtainable by reaction of sulphuryl chloride with a mixture of the alcohols R¹OH, R²OH and R³OH, where R¹, R² and R³ in these alcohols have the meanings mentioned for the general formula 1, except that Y is exclusively hydrogen and Z is either hydrogen, methyl or ethyl.

2. Mixtures of sulphuric esters according to Claim 1, **characterized in that**
R¹ is an aliphatic radical having 4-30 carbon atoms,
R² is a radical of the general formula 2 where
n is an integer from 0-10,
m is an integer from 1-10,
X is an aliphatic radical having 12-24 carbon atoms and
Y is H or SO₂(OM), where M represents H, alkali metal, ammonium, mono-, di-, tri- or tetra-(C₁-C₆-alkyl)ammonium ions or mono-, di-, tri- or tetra-(C₂-C₆-alkanol)ammonium ions, and
R³ is a radical of the general formula 3 where
m is an integer from 3 - 35,
R⁴ is H or methyl and
Z is H, methyl, ethyl or SO₂(OM), where M independently represents H, alkali metal, ammonium, mono-, di-, tri- or tetra-(C₁-C₆-alkyl)ammonium ions or mono-, di-, tri- or tetra- (C₂₋C₆-alkanol)ammonium ions, and
a, b and c are identical or different and 0, 1 or 2 and a+b+c=2.

3. Mixtures of sulphuric esters according to Claim 1, **characterized in that**
R¹ is an aliphatic radical having 8-20 carbon atoms,
R² is a radical of the general formula 2 where
n is an integer from 0 - 5,
m is an integer from 1 - 5,
X is an aliphatic radical having 16-22 carbon atoms and
Y is H,
R³ is a radical of the general formula 3 where
p is an integer from 9-22,
R⁴ is H and
Z is H and
a, b and c are identical or different and 0, 1 or 2 and a+b+c=2.

4. Process for preparing mixtures of sulphuric esters according to one or more of Claims 1 to 3, **characterized in that** a mixture of the alcohols R¹OH, R²OH and R³OH, where R¹, R² and R³ in these alcohols have the meanings indicated for the general formula 1, but Y is exclusively hydrogen and Z is either hydrogen, methyl or ethyl, is reacted with sulphuryl chloride.

5. Process according to Claim 4, **characterized in that** 3 mol of the mixture of the alcohols R¹OH, R²OH and R³OH are reacted with 1.5-2.5 mol, preferably 1.5-1.8 mol, of sulphuryl chloride.

6. Process according to Claim 4 or 5, **characterized in that** the alcohols R¹OH, R²OH and R³OH are used in the following amount ratios:
| | |
|---|---|
| R¹OH | 10 - 40, preferably 30 - 40, mol% |
| R²OH | 20 - 80, preferably 30 - 40, mol% and |
| R³OH | 10 - 40, preferably 30 - 40, mol%, |
the amounts of the three alcohols always adding up to 100 mol%.

7. Sulphuric esters of the general formula 1 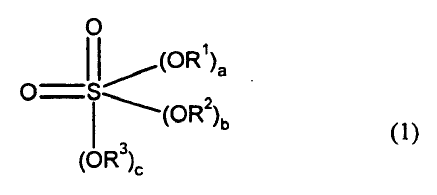 where
R¹, R² and R³ have the meanings mentioned for the mixtures of the sulphuric esters in Claim 1 and
a, b and c are identical or different and 0 or 1 and a+b+c=2.

8. Sulphuric esters according to Claim 7, **characterized in that**
R¹, R² and R³ have the meanings mentioned for the mixtures of the sulphuric esters in Claim 2 and
a, b and c are identical or different and 0 or 1 and a+b+c=2.

9. Sulphuric esters according to Claim 7, **characterized in that**
R¹, R² and R³ have the meanings mentioned for the mixtures of the sulphuric esters in Claim 3 and
a, b and c are identical or different and 0 or 1 and a+b+c=2.

10. Organic or aqueous-organic formulations including 25 - 70% by weight of the mixtures of the sulphuric esters according to one or more of Claims 1-3 or of the sulphuric esters according to one or more of Claims 7-9.

11. Organic or aqueous-organic formulations according to Claim 10, **characterized in that** the organic solvents used are mono-, di- or oligoethylene glycols, -propylene glycols or -ethylene/propylene glycols, their mono- or diethers or mixtures thereof, especially butyldiglycol or methyldiglycol.

12. Use of the mixtures of the sulphuric esters according to one or more of Claims 1-3, of the sulphuric esters according to one or more of Claims 7-9 or of the formulations according to Claim 10 or 11 as dyeing auxiliaries in the dyeing of nitrogenous fibre materials, preferably wool.

13. Use according to Claim 12, wherein the dyeing is carried out with acid dyes, 1:1 metal complex dyes, 1:2 metal complex dyes, chrome dyes or mixtures thereof.

## Revendications

1. Mélange d'esters d'acide sulfurique de formule générale 1, 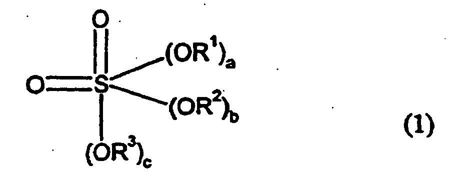 dans laquelle
R¹ représente un radical aliphatique ayant de 1 à 30 atomes de carbone,
R² représente un radical de formule générale 2, 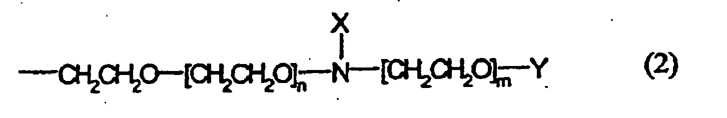 dans laquelle
n représente un entier de 0 à 30,
m représente un entier de 1 à 29,
X représente un radical aliphatique ayant de 4 à 24 atomes de carbone, et
Y représente un H ou un SO₂(OM), dans lequel M représente indépendamment les uns des autres un H, un métal alcalin, un ammonium, un ion mono-, di-, tri- ou tétra-(C₁-C₆-alkyl)ammonium ou un ion mono-, di-, tri- ou tétra-(C₂-C₆₋alcanol)ammonium,
R³ représente un radical de formule générale 3, 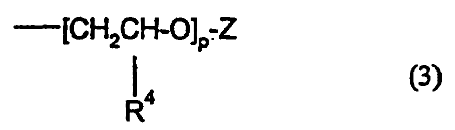 dans laquelle
p représente un entier de 4 à 35,
R⁴ représente un H, un méthyle, un éthyle ou un phényle, ou des mélanges de H et de méthyle, et
Z représente un H, un méthyle, un éthyle ou un SO₂(OM), dans lequel M représente indépendamment les uns des autres un H, un métal alcalin, un ammonium, un ion mono-, di-, tri- ou tétra-(C₁-C₆-alkyl)ammonium ou un ion mono-, di-, tri-ou tétra-(C₂-C₆-alcanol)ammonium,
a, b et c sont identiques ou différents et valent 0, 1 ou 2, et a+b+c=2,
pouvant être obtenu par réaction du chlorure de sulfuryle avec un mélange des alcools R¹OH, R²OH et R³OH, où dans ces alcools, R¹, R² et R³ présentent les significations mentionnées pour la formule générale 1, mais Y représente exclusivement un hydrogène et Z représente soit un hydrogène, soit un méthyle, soit un éthyle.

2. Mélanges d'esters d'acide sulfurique selon la revendication 1, **caractérisés en ce que**
R¹ représente un radical aliphatique ayant de 4 à 30 atomes de carbone,
R² représente un radical de formule générale 2, dans laquelle
n représente un entier de 0 à 10,
m représente un entier de 1 à 10,
X représente un radical aliphatique ayant de 12 à 24 atomes de carbone, et
Y représente un H ou un SO₂(OM), dans lequel M représente indépendamment les uns des autres un H, un métal alcalin, un ammonium, un ion mono-, di-, tri- ou tétra-(C₁-C₆-alkyl)ammonium ou un ion mono-, di-, tri- ou tétra-(C₂-C₆₋alcanol)ammonium, et
R³ représente un radical de formule générale 3, dans laquelle
m représente un entier de 3 à 35,
R⁴ représente un H ou un méthyle, et
Z représente un H, un méthyle, un éthyle ou un SO₂(OM), dans lequel M représente indépendamment les uns des autres un H, un métal alcalin, un ammonium, un ion mono-, di-, tri- ou tétra-(C₁-C₆-alkyl) ammonium ou un ion mono-, di-, tri-ou tétra-(C₂-C₆-alcanol) ammonium,
a, b et c sont identiques ou différents et valent 0, 1 ou 2, et a+b+c=2.

3. Mélanges d'esters d'acide sulfurique selon la revendication 1, **caractérisés en ce que**
R¹ représente un radical aliphatique ayant de 8 à 20 atomes de carbone,
R² représente un radical de formule générale 2, dans laquelle
n représente un entier de 0 à 5,
m représente un entier de 1 à 5,
X représente un radical aliphatique ayant de 16 à 22 atomes de carbone, et
Y représente un H,
R³ représente un radical de formule générale 3, dans laquelle
p représente un entier de 9 à 22,
R⁴ représente un H, et
Z représente un H, et
a, b et c sont identiques ou différents et valent 0, 1 ou 2, et a+b+c=2.

4. Procédé de préparation de mélanges d'esters d'acide sulfurique selon l'une ou plusieurs des revendications 1 à 3, **caractérisé en ce qu'**un mélange des alcools R¹OH, R²OH et R³OH est mis à réagir avec du chlorure de sulfuryle, où dans ces alcools, R¹, R² et R³ présentent les significations indiquées pour la formule générale 1, mais Y représente exclusivement un hydrogène et Z représente soit un hydrogène, soit un méthyle, soit un éthyle.

5. Procédé selon la revendication 4, **caractérisé en ce que** 3 mol du mélange des alcools R¹OH, R²OH et R³OH sont mis à réagir avec de 1,5 à 2,5 mol, de préférence de 1,5 à 1,8 mol de chlorure de sulfuryle.

6. Procédé selon la revendication 4 ou 5, **caractérisé en ce que** les alcools R¹OH, R²OH et R³OH sont utilisés dans les proportions suivantes
R¹OH de 10 à 40, de préférence de 30 à 40 % en moles,
R²OH de 20 à 80, de préférence de 30 à 40 % en moles, et
R³OH de 10 à 40, de préférence de 30 à 40 % en moles,
où la somme des quantités des trois alcools est toujours de 100 % en moles.

7. Ester d'acide sulfurique de formule générale 1, 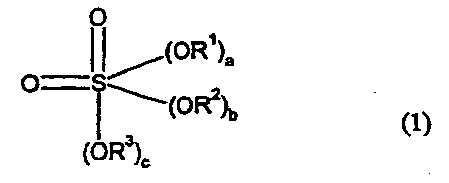 dans laquelle
R¹, R² et R³ présentent les significations mentionnées dans la revendication 1 pour les mélanges des esters d'acide sulfurique, et
a, b et c sont identiques ou différents et valent 0 ou 1, et a+b+c=2.

8. Ester d'acide sulfurique selon la revendication 7, **caractérisé en ce que**
R¹, R² et R³ présentent les significations mentionnées dans la revendication 2 pour les mélanges des esters d'acide sulfurique et
a, b et c sont identiques ou différents et valent 0 ou 1, et a+b+c=2.

9. Ester d'acide sulfurique selon la revendication 7, **caractérisé en ce que**
R¹, R² et R³ présentent les significations mentionnées dans la revendication 3 pour les mélanges des esters d'acide sulfurique et
a, b et c sont identiques ou différents et valent 0 ou 1, et a+b+c=2.

10. Formulations organiques ou aqueuse-organiques contenant de 25 à 70 % en poids des mélanges des esters d'acide sulfurique selon l'une ou plusieurs des revendications 1 à 3 ou des esters d'acide sulfurique selon l'une ou plusieurs des revendications 7 à 9.

11. Formulations organiques ou aqueuse-organiques selon la revendication 10, **caractérisées en ce que** l'on utilise, en tant que solvants organiques, des mono-, des di- ou des oligoéthylèneglycols, -propylèneglycols ou -éthylène/propylèneglycols, leurs mono- ou diéthers ou leurs mélanges, en particulier le butyldiglycol ou le méthyldiglycol.

12. Utilisation des mélanges des esters d'acide sulfurique selon l'une ou plusieurs des revendications 1 à 3, des esters d'acide sulfurique selon l'une ou plusieurs des revendications 7 à 9 ou des formulations selon la revendication 10 ou 11 en tant qu'auxiliaire de teinture pour la teinture de matières fibreuses azotées, de préférence de la laine.

13. Utilisation selon la revendication 12, dans laquelle la teinture est réalisée avec des colorants acides, des colorants à complexe métallique 1:1, des colorants à complexe métallique 1:2, des colorants au chrome ou leurs mélanges.
